# EUROPEAN PATENT SPECIFICATION

(11) **EP 0 570 383 B1**
(45) Date of publication and mention of the grant of the patent: **05.07.1995**
(21) Application number: 92901533.7
(22) Date of filing: 10.12.1991
(51) Int. Cl.: C07C 227/08

(54) **PROCESS FOR PREPARING SOLID BETAINES**
Verfahren zur Herstellung von festen Betainen
PROCEDE DE PREPARATION DE BETAINES SOLIDES

(30) Priority: 08.02.1991 US 652617; 08.02.1991 US 652616; 01.07.1991 US 723976; 01.07.1991 US 723919
(43) Date of publication of application: 24.11.1993
(73) Proprietor: ALBEMARLE CORPORATION, Richmond, Virginia 23219 (US)
(72) Inventor: PERINE, Jeffrey, W., Baton Rouge, LA 70810 (US); SMITH, Kim, R., Baton Rouge, LA 70810 (US); SAUER, Joe, D., Baton Rouge, LA 70816 (US); BORLAND, James, E., Baton Rouge, LA 70815 (US)
(74) Representative: Schwabe - Sandmair - Marx
(86) International application number: US9109274
(87) International publication number: WO9213825

(56) References cited:
- EP-A- 0 353 580
- GB-A- 1 185 111
- US-A- 2 082 275
- US-A- 2 464 260
- US-A- 3 555 079
- US-A- 4 824 867

## Description

### Field of Invention

This invention relates to a process for preparing solid betaines from tert-amines.

### Background

As disclosed in British Patent 1,185,111 (Morris) and U.S. Patents 2,082,275 (Daimler et al.), 3,555,079 (Marumo et al.), and 4,832,871 (Bade), it is known that tert-amines can be quaternized with haloalkanoate salts in water or a polar aprotic solvent to prepare betaines in solution form, most commonly as 30-35% active aqueous solutions.

Nandakumar et al., *Journal of the Oil Technologists' Association of India,* Volume 11(2), pp. 31-34(1979) show that it is also known that a betaine solution can be obtained by reacting the tert-amine with a haloalkanoate ester to form a quaternary ammonium ester and then reacting the intermediate with a base to convert it to the corresponding betaine.

Solid betaines have the advantages over betaine solution that they can be transported at lower costs and offer more flexibility in the formulation of products from betaines. It is possible to recover solid betaines from the solutions described above, but it would be preferable to be able to prepare the betaines directly in solid form.

### Summary of Invention

It has now been found that solid betaines can be produced by the reaction of (1) a haloalkanoate salt with a tert-amine or (2) a base with the quaternary ammonium ester resulting from the quaternization of a tert-amine with an alkyl haloalkanoate when the reaction is conducted in a liquefied gas solvent or in a polar aprotic solvent in which the betaine is substantially insoluble.

### Detailed Description

Except for the use of a reaction medium from which the product can be easily recovered in solid form, the reactions of the invention can be conducted by conventional means.

As in the known processes, the tert-amine which is reacted with the haloalkanoate may be any of a variety of tert-amines, e.g., any of the tert-amines of Morris, Daimler et al., Marumo et al., or Bade. However, those which are generally the most valuable to employ are the tert-amines in which at least one of the N-substituents is an alkyl or hydroxyalkyl group and the remaining N-substituents are aliphatic or cyclic organic groups which may be hydrocarbyl or non-hydrocarbyl in nature, e.g., alkyl, hydroxyalkyl, polyoxyethylene, alkylamidoalkyl, phenyl, or benzyl, including those in which an alkyl or hydroxyalkyl group is attached to a nitrogen which is a member of a heterocyclic ring, such as a morpholine ring.

Among the preferred tert-amines are the compounds corresponding to the formula RR'R''N in which R is a linear or branched-chain alkyl group containing 6-22 carbons, more preferably a primary alkyl group containing 8-18 carbons; R' is methyl, ethyl, or 2-hydroxyethyl; and R'' is independently selected from methyl, ethyl, 2-hydroxyethyl, and linear and branched-chain alkyl groups containing 6-22 carbons. These tert-amines may be used alone or in combination.

The most preferred of these tert-amines are those in which at least a majority of alkyl groups in the tert-amine or tert-amine mixture are linear and R' and R'' are independently selected from methyl, ethyl, and 2-hydroxyethyl, especially those in which both R' and R'' are methyl.

Depending on whether the tert-amine is to be converted to a betaine directly or indirectly, the haloalkanoate with which it is reacted is a salt or an ester.

When the haloalkanoate is a salt, it is generally a Group IA or IIA metal salt or ammonium salt of an ω-haloalkanoic acid in which the halo substituent is chloro, bromo, or iodo. Neither the size nor the degree of linearity of the alkanoic moiety is critical, but it is most commonly a moiety containing up to about 30 carbons and in which any branching is confined to carbons other than the carbon to which the halo substituent is attached, since any branching on that carbon could be expected to slow the reaction significantly.

Exemplary of the haloalkanoate salts that can be used are the sodium, potassium, lithium, rubidium, cesium, beryllium, magnesium, calcium, strontium, barium, and ammonium salts of chloroacetic, chloropropionic, chlorobutyric, chloropentanoic, chlorohexanoic, chloroheptanoic, chloro-β-ethylhexanoic, and corresponding bromo- and iodoalkanoic acids. The preferred haloalkanoates are compounds corresponding to the formula X(CH₂)ₙCOOM in which X is chloro, bromo, or iodo; M is alkali metal or ammonium; and n is an integer of 1-6. As in conventional processes, the most preferred haloalkanoate salt is sodium chloroacetate.

When the haloalkanoate is an ester, it is an alkyl ester of any of the ω-haloalkanoic acids mentioned above, e.g., a methyl, ethyl, propyl, isopropyl, butyl, isobutyl, pentyl, hexyl, octyl, 2-ethylhexyl, decyl, dodecyl, eicosyl, or triacontyl ester. Neither the size nor the degree of linearity of the alkyl or alkanoic moiety is critical, so the esters include compounds in which both moieties are large or small or in which one is small and the other large and in which both moieties are linear or branched or in which one is linear and the other branched. However, most commonly both the alkyl and the alkanoic moieties are moieties containing up to about 30 carbons; and, as in the salts, it is preferred that any branching in the alkanoic moiety be confined to carbons other than the carbon to which the halo substituent is attached.

The preferred haloalkanoate esters are compounds corresponding to the formula X(CH₂)ₙCOOZ in which X is chloro, bromo, or iodo, Z is a linear or branched-chain alkyl group containing 1-6 carbons; and n is an integer of 1-6. Ordinarily, the most preferred alkyl haloalkanoates are the methyl and ethyl chloroacetates.

Regardless of whether the haloalkanoate is a salt or an ester, the amount employed to quaternize the tert-amine is usually at least the stoichiometic amount. However, it is preferred not to use too much of the haloalkanoate reactant, so it can be most preferable to utilize the reactants in substantially stoichometric amounts.

### Tert-amine/haloalkanoate salt

The reaction between the tert-amine and a haloalkanoate salt may be conducted in a liquefied gas or in a polar aprotic solvent.

### Part A - Polar aprotic solvent

When the reaction medium is a polar aprotic solvent, it may be any such organic solvent in which the betaine is substantially insoluble, at least at room temperature. Such solvents include, e.g., ketones such as 2-propanone, 2-butanone, 3-methyl-2-butanone, 2-pentanone, and 3-pentanone; nitriles such as acetonitrile, propionitrile, butyronitrile, and isobutyronitrile; cyclic ethers such as tetrahydrofuran, tetrahydropyran, and dioxane; sulfoxides such as dimethylsulfoxide; and amides such as hexamethylphosphoramide, N-methylpyrrolidone, dimethylformamide, dimethylacetamide, and diethylformamide. However, the preferred solvents are the esters.

Esters which may be used in the process include aliphatic, cycloaliphatic, and aromatic esters, such as the methyl, ethyl, propyl, isopropyl, butyl, isobutyl, t-butyl, pentyl, hexyl, octyl, decyl, dodecyl, cyclohexyl, phenyl, tolyl, and benzyl esters of formic, acetic, propionic, butyric, isobutyric, pentanoic, hexanoic, heptanoic, octanoic, and benzoic acids. The preferred esters are ordinarily the alkyl alkanoates in which both the alkyl and the alkanoic moieties contain 2-6 carbons, especially ethyl acetate.

When the reaction is effected in a polar aprotic solvent, the process is conducted by combining the tert-amine with at least a portion of the haloalkanoate salt in the solvent and allowing the quaternization reaction to occur. It is generally preferred to include all of the haloalkanoate in the initial reaction mixture, but satisfactory results can also be obtained when the salt is gradually added in increments. If desired, some preformed betaine product may also be included in the initial reaction mixture to shorten the time before which any noticeable quaternization occurs.

Although the reaction can be effected at room temperature and atmospheric or subatmospheric presure, it is ordinarily preferred to use an elevated temperature and/or superatmospheric pressure to speed the reaction. The preferred temperatures are in the range of 50-150° C, most conveniently the reflux temperatures of the reaction mixtures; and the preferred pressures are 0.1-2.1 MPa. The time required for the reaction depends, of course, on the temperature and pressure used, varying from weeks at room temperature and atmospheric pressure to only minutes at 150° C and 0.4 MPa.

The quaternization may be accomplished in a batch process, but a continuous process is apt to be preferred, and any unreacted haloalkanoate remaining in the reaction mixture at the end of the reaction may be recovered and recycled.

Because of the insolubility of the betaine product in the reaction medium, it is easily recovered as a solid by filtration. The solid thus recovered is a betaine product having high activity; and its activity can be increased to an even higher level, in fact to as high as 100%, by removing some-to-all of the by-product salt and any remaining solvent, if desired.

Actually, it is not necessarily desirable to remove the solvent and by-product salt that remain with the solid betaine after filtration, since the amount of residual solvent is minimal, and the effect that the salt could have in increasing the viscosity of solutions later formed from the betaine could be advantageous in some instances and disadvantageous in others. However, when the objective is to obtain a substantially pure betaine, it is beneficial to separate the betaine and salt by solvent extraction and to supplement the filtration with vacuum drying to remove the remaining solvent.

In a preferred embodiment of the invention, the betaine is recovered by cooling the reaction mixture at the end of the reaction; filtering to separate the betaine and salt from the solvent; washing the salt-contaminated betaine with a solvent of the same type used in the reaction, i.e., a non-solvent for the betaine and by-product salt; and then washing with a liquid, such as isopropanol, which is a solvent for the betaine but not the salt.

### Part B - Liquefied gas solvent

When the reaction medium for the tert-amine/haloalkanoate salt reaction is a liquefied gas, it may be the liquefied form of any normally gaseous material which is inert in the sense that it will neither prevent the reaction from occurring nor react with the product. Such normally gaseous materials include, e.g., air, oxygen, carbon dioxide, nitrogen, argon, ethylene, methane, ethane, propane, butane, isobutane, trifluoromethane, tetrafluoromethane, chlorotrifluoromethane, and mixtures thereof. Carbon dioxide is sometimes preferred.

Most commonly, the liquefied gas which is employed is one that is commercially available and can simply be introduced into the reaction vessel in liquid form and maintained in liquid form by the use of pressure. However, if desired, it may be acquired in the gaseous state and introduced into the reaction vessel via a compressor to liquefy it.

Because of the greater expense involved in liquefying a gas which has a very low critical temperature, it is frequently preferred to employ as the liquefied gas a normally gaseous material which has a critical temperature that is above or not much below room temperature, generally a critical temperature of at least 0° C, preferably at least 20° C, e.g., materials such as ethylene, carbon dioxide, chlorotrifluoromethane, ethane, propane, butane, and isobutane.

The process is conducted by combining the tert-amine with at least a portion of the haloalkanoate salt in the liquefied gas solvent and allowing the quaternization reaction to occur. It is generally preferred to include all of the haloalkanoate and all of the solvent in the initial reaction mixture, but satisfactory results can also be obtained when the salt is gradually added in increments and/or a portion of the solvent is fed into the reactor after the reaction has commenced. If desired, some preformed betaine product may also be included in the initial reaction mixture to shorten the time before which any noticeable quaternization occurs.

Although the reaction can be effected at room temperature, it is ordinarily preferred to use an elevated temperature, preferably a temperature in the range of 50-150° C, to speed the reaction. The process is conducted under a pressure sufficient to maintain the liquefied gas in the liquid state, and pressures in excess of the minimum requirements can be used if desired. Most commonly, the amount of pressure used is chosen to be consistent with conducting an economical process, usually a pressure in the range of about 4.9-8.5 MPa.

In general, the reaction may be conducted so as to have supercritical or subcritical conditions.

The quaternization may be accomplished by a batch, semi-batch, or continuous process in the presence of the liquefied gas, which is vented from the reaction vessel after completion of the reaction. Because of the use of the liquefied gas as the sole solvent, the solid betaine product remaining after the venting of the system can be recovered without the need for the crystallization, centrifugation, and drying steps required in earlier betaine syntheses unless it is desired to remove the salts that remain with the product. Thus the process is an economically-advantageous method of preparing solid betaines.

### Tert-amine /haloalkanoate ester

The reaction between the tert-amine and a haloalkanoate ester is generally conducted in the same liquefied gas or polar aprotic solvent which it is desired to use in the subsequent reaction with a base, although (1) it may sometimes be desirable to use a liquefied gas for the quaternization reaction and a polar aprotic solvent for the base reaction and (2) a liquid solvent different from the polar aprotic solvent of the base reaction may be employed for the quaternization when it is intended to recover the quaternary ammonium ester from its reaction mixture before reacting it with the base. The conditions utilized for the reaction are essentially the same as those used in the tert-amine/haloalkanoate salt quaternization described above.

### Part A - Polar aprotic solvent

When the resultant quaternary ammonium ester is to be recovered from its synthesis reaction mixture before being subjected to reaction with the base, the reaction medium may be any of the polar aprotic solvents mentioned above in the discussion of the tert-amine/haloalkanoate salt reaction. However:
(1) regardless of whether the ester is to be recovered from its reaction mixture before being reacted with the base or the base reaction is to be effected in the ammonium ester synthesis mixture, it is desirable for the solvent to be one in which the quaternary ammonium ester and its corresponding betaine are substantially insoluble, at least at room temperature, and
(2) since the efficiency of the betaine synthesis would be considerably reduced if a reactive solvent were used for the reaction between the quaternary ammonium ester and the base, only those solvents which would be inert in such a reaction should be used in that reaction or in the quaternary ammonium ester synthesis when the base reaction is to be effected in the reaction mixture resulting from the synthesis of the ester.

Thus, the amides, nitriles, and esters, which would not be inert in the base reaction, should not be used therein; but the other aforementioned solvents are quite suitable. The preferred solvents are the ketones, especially 2-butanone.

### Part B - Liquefied gas solvent

When the quaternization of the tert-amine with the haloalkanoate ester is effected in a liquefied gas, the solvent may be any of the liquefied gases mentioned above. After completion of the quaternization reaction, the system is vented to remove the liquefied gas when it is desired to hydrolyze the quaternary ammonium ester in an organic solvent, or a base is added to the system when it is desired to conduct both steps of the process in the liquefied gas solvent.

### Ammonium ester/base

As already indicated, the quaternary ammonium ester which is contacted with a base to form a betaine in the practice of the invention may be (1) the reaction mixture resulting from the synthesis of the ester in a suitable polar aprotic solvent, (2) a mixture of such a solvent with a quaternary ammonium ester which has been (a) recovered from such a reaction mixture or (b) prepared in a liquefied gas solvent which has been vented from the system, or (3) the unvented reaction mixture resulting from the synthesis of the ester in a liquefied gas.

Processes in which a liquefied gas is used as the solvent for both the quaternization and the hydrolysis steps have a certain economic advantage. However:
(1) they can limit the choices as to liquefied gases which may be used, since a liquefied gas which would be inert to the quaternization reaction would not necessarily permit the hydrolysis to occur, and
(2) the use of a liquefied gas rather than an organic solvent for the hydrolysis reaction leaves the betaine product contaminated with salt and alcohol by-products which might have to be removed by solvent extraction or evaporation, e.g., when the by-products would detrimentally affect the properties of the betaine in a particular application and/or when an alcohol by-product would solubilize a substantial amount of the product.

Thus, it is often preferable to conduct the hydrolysis in a suitable polar aprotic solvent, regardless of the reaction medium used for the quaternization reaction.

The base employed for the reaction may be any of the bases conventionally used in such reactions, usually aluminum hydroxide or the hydroxide of a Group IA or IIA metal, such as lithium, sodium, potassium, rubidium, cesium, beryllium, magnesium, calcium, strontium, or barium. It is ordinarily used in the stoichiometric amount to effect complete reaction, and the preferred base is generally sodium hydroxide.

Although the base may be utilized in virtually any form, e.g., as a powder, pellets, or aqueous or alcoholic solution, the more dilute solutions, i.e., those containing less than about 25% of the base, are not very desirable for use in the process because of the objective of preparing the betaine in solid form. The large amount of water contributed by a dilute aqueous solution of the base could solubilize the betaine and/or cause foaming if later concentration were to become necessary.

### Part A - Polar aprotic solvent

When the hydrolysis is conducted in a polar aprotic solvent, it is critical that the solvent be one in which the betaine is substantially insoluble, at least at room temperature, e.g., the solvents mentioned above. The reaction of the quaternary ammonium ester with the base can be effected at room temperature and atmospheric or subatmospheric pressure, but it is ordinarily preferred to use an elevated temperature and/or superatmospheric pressure to speed the reaction. The preferred temperatures are in the range of 50-150° C, most conveniently the reflux temperatures of the reaction mixtures; and the preferred pressures are 0.1-2.1 MPa. Because of the insolubility of the betaine product in the reaction solvent, it is easily recovered as a solid by filtration.

### Part B - Liquefied gas solvent

When the hydrolysis is conducted in a liquefied gas, the solvent may be any of the liquefied gases mentioned above. The hydrolysis can be effected at room temperature but is preferably conducted at a higher temperature, e.g., 50-150° C; and it is conducted under a pressure sufficient to maintain the liquefied gas in the liquid state, preferably a pressure of 4.9-8.5 MPa. When the reaction is complete, the product is easily recovered by venting the system.

Regardless of which reaction medium is used for the hydrolysis, the solid recovered is a betaine product having high activity; and its activity can be increased to an even higher level, in fact to as high as 100%, by removing some-to-all of the salt by-product of the base reaction and any remaining solvent, if desired.

The invention is advantageous as a convenient means of preparing solid betaines which can be used in the same applications as conventional betaine solutions, e.g., in the production of soaps and shampoos, but which offer more flexibility in the formulation of products because of not having any solvent associated therewith and which are also more economical to transport.

The following examples are given to illustrate the invention and are not intended as a limitation thereof.

### EXAMPLE I

A suitable reaction vessel was charged with 153g of N-hexadecyldimethylamine, 54g of sodium chloroacetate, 15.4g of preformed betaine derived from the same reactants, and 300 mL of ethyl acetate. The reaction mixture was heated to reflux with stirring and refluxed for eight hours, after which it was cooled and vacuum-filtered to separate the solid betaine/sodium chloride mixture from the solvent and unreacted amine and sodium chloroacetate. A sample of the crude betaine was partially dissolved in refluxing ethyl acetate, gravity-filtered to remove the sodium chloride, and recrystallized to yield 95% active betaine as a tacky, off-white solid.

### EXAMPLE II

A suitable reaction vessel was charged with 100g of N-tetradecyldimethylamine, 53g of ethyl chloroacetate, and 500 mL of 2-butanone. The reaction mixture was heated to reflux with stirring and refluxed for eight hours to quaternize the amine. Then 50g of 98% active sodium hydroxide pellets were added to the mixture, and stirring with reflux was continued for an additional hour. HPLC analysis showed that conversion of the quaternary ammonium ester to betaine was complete, and the mixture was filtered to remove inorganic material, cooled in an ice bath, and filtered again to provide the betaine product as a white solid.

### EXAMPLE III

A suitable reaction vessel was charged with 0.20 mol of N-hexadecyldimethylamine, 0.21 mol of methyl chloroacetate, and 200 mL of 2-butanone. The reaction mixture was heated to reflux with stirring and refluxed for six hours to quaternize the amine. Then 12.5 M aqueous sodium hydroxide was added dropwise over five minutes to introduce 0.22 mol of sodium hydroxide. The resultant mixture was stirred at reflux (80° C) for ten minutes, cooled to 40° C, and vacuum-filtered through Whatman #1 paper to remove the sodium chloride by-product. The filtrate was chilled to below 0° C to precipitate the betaine, which was then collected by vacuum filtration through Whatman #2 paper and dried on a vacuum pump overnight to yield 45.4g of N-hexadecyl-N,N-dimethylglycine as a white powder.

## Claims

1. A process for preparing a solid betaine by the reaction of (1) a haloalkanoate salt with a tert-amine or (2) a base with the quaternary ammonium ester resulting from the quaternization of a tert-amine with an alkyl haloalkanoate, characterized in that the reaction is conducted in a liquefied gas solvent or in a polar aprotic solvent in which the solid betaine is substantially insoluble.

2. The process of claim 1 wherein a base is reacted with the quaternary ammonium ester resulting from the quaternization of a tert-amine with an alkyl haloalkanoate.

3. The process of claim 2 wherein both the quaternization and the base reactions are conducted in a polar aprotic solvent in which the solid betaine is substantially insoluble.

4. The process of claim 3 wherein the quaternary ammonium ester which is reacted with the base is an ester which has been recovered from its synthesis reaction mixture before being contacted with the base.

5. The process of claim 3 wherein the tert-amine is reacted with the haloalkanoate to form the quaternary ammonium ester in the polar aprotic solvent, and the base is then added to the resultant mixture and reacted with the quaternary ammonium ester.

6. The process of claim 2 wherein both the quaternization and the base reactions are conducted in a liquefied gas as the solvent.

7. The process of claim 2 wherein the quaternization reaction is conducted in a liquefied gas solvent and the base reaction is conducted in a polar aprotic solvent in which the solid betaine is substantially insoluble.

8. The process of any of claims 2-7 wherein (a) the base is a Group IA or IIA metal hydroxide, (b) the haloalkanoate is a compound corresponding to the formula X(CH₂)ₙCOOZ in which X is chloro, bromo, or iodo; Z is an alkyl group containing 1-6 carbons, and (c) the tert-amine is a compound corresponding to the formula RR'R''N in which R is an alkyl group containing 6-22 carbons; R' is methyl, ethyl, or 2-hydroxyethyl; and R'' is independently selected from methyl, ethyl, 2-hydroxyethyl, and alkyl groups containing 6-22 carbons.

9. The process of claim 8 wherein (a) the base is sodium hydroxide, (b) the haloalkanoate is ethyl chloroacetate and (c) R is a primary alkyl group containing 8-18 carbons and R' and R'' are independently selected from methyl and primary alkyl groups containing 8-18 carbons.

10. The process of claim 1 wherein a haloalkanoate salt is reacted with a tert-amine in a polar aprotic solvent in which the solid betaine is substantially insoluble.

11. The process of claim 10 wherein (a) the haloalkanoate is a compound corresponding to the formula X(CH₂)ₙCOOM in which X is chloro, bromo, or iodo; M is alkali metal or ammonium; and n is an integer of 1-6, (b) the tert-amine is a compound corresponding to the formula RR'R''N in which R is an alkyl group containing 6-22 carbons; R' is methyl, ethyl, or 2-hydroxyethyl; and R'' is independently selected from methyl, ethyl, 2-hydroxyethyl, and alkyl groups containing 6-22 carbons, and (c) the solvent is an ester, ketone, ether, amide, nitrile, or sulfoxide.

12. The process of claim 11 wherein (a) the haloalkanoate is sodium chloroacetate, (b) R is a primary alkyl group containing 8-18 carbons and R' and R'' are independently selected from methyl and primary alkyl groups containing 8-18 carbons, and (c) the solvent is an ester.

13. The process of claim 1 wherein a haloalkanoate salt is reacted with a tert-amine in a liquefied gas as the solvent.

14. The process of claim 13 wherein (a) the haloalkanoate is a compound corresponding to the formula X(CH₂)ₙCOOM in which X is chloro, bromo, or iodo; M is alkali metal or ammonium; and n is an integer of 1-6, (b) the tert-amine is a compound corresponding to the formula RR'R''N in which R is an alkyl group containing 6-22 carbons; R' is methyl, ethyl, or 2-hydroxyethyl; and R'' is independently selected from methyl, ethyl, 2-hydroxyethyl, and alkyl groups containing 6-22 carbons, and (c) the solvent is a liquefied gas which has a critical temperature of at least 0° C.

15. The process of claim 13 wherein (a) the haloalkanoate is sodium chloroacetate, (b) R is a primary alkyl group containing 8-18 carbons and R' and R'' are independently selected from methyl and primary alkyl groups containing 8-18 carbons, and (c) the liquefied gas is carbon dioxide.

## Patentansprüche

1. Verfahren zur Herstellung eines festen Betains durch die Reaktion von (1) einem Halogenalkanoatsalz mit einem tertiären Amin oder (2) einer Base mit dem quaternären Ammoniumester, der aus der Quaternisierung eines tertiären Amins mit einem Alkylhalogenalkanoat entsteht, dadurch gekennzeichnet, daß die Reaktion in einem Lösungsmittel aus einem verflüssigten Gas oder einem polaren aprotischen Lösungsmittel durchgeführt wird, in dem das feste Betain im wesentlichen unlöslich ist.

2. Verfahren nach Anspruch 1, bei dem eine Base mit dem aus der Quaternisierung eines tertiären Amins mit einem Alkylhalogenalkanoat resultierenden quaternären Ammoniumester zur Umsetzung gebracht wird.

3. Verfahren nach Anspruch 2, bei dem sowohl die Quaternisierung als auch die Umsetzung mit der Base in einem polaren aprotischen Lösungsmittel durchgeführt werden, in dem das feste Betain im wesentlichen unlöslich ist.

4. Verfahren nach Anspruch 3, bei dem der mit der Base umgesetzte quaternäre Ammoniumester ein Ester ist, der aus seiner Synthesereaktionsmischung gewonnen wird, ehe man ihn mit der Base in Kontakt bringt.

5. Verfahren nach Anspruch 3, bei dem das tertiäre Amin mit dem Halogenalkanoat zur Umsetzung gebracht wird, um den quaternären Ammoniumester im polaren aprotonischen Lösungsmittel herzustellen, und die Base der dabei entstehenden Mischung dann zugesetzt und mit dem quaternären Ammoniumester zur Umsetzung gebracht wird.

6. Verfahren nach Anspruch 2, bei dem sowohl die Quaternisierung als auch die Umsetzung mit der Base in einem verflüssigten Gas als Lösungsmittel durchgeführt werden.

7. Verfahren nach Anspruch 2, bei dem die Quaternisierungsreaktion in einem Lösungsmittel aus verflüssigtem Gas und die Umsetzung mit der Base in einem polaren aprotischen Lösungsmittel durchgeführt wird, in dem das feste Betain im wesentlichen unlöslich ist.

8. Verfahren nach einem der Ansprüche 2 bis 7, bei dem (a) die Base ein Metallhydroxid der Gruppe IA oder IIA ist (b) das Halogenalkanoat eine Verbindung der Formel X(CH₂)ₙCOOZ ist, in der X Chlor, Brom oder Iod und Z eine Alkylgruppe mit 1 bis 6 Kohlenstoffatomen bedeutet, und (c) das tertiäre Amin eine Verbindung der Formel RR'R''N ist, in der R eine Alkylgruppe mit 6 bis 22 Kohlenstoffatomen, R' Methyl, Ethyl oder 2-Hydroxyethyl und R'' unabhängig aus Methyl, Ethyl, 2-Hydroxyethyl und Alkylgruppen mit 6 bis 22 Kohlenstoffatomen ausgewählt ist.

9. Verfahren nach Anspruch 8, bei dem (a) die Base Natriumhydroxid ist, (b) das Halogenalkanoat Ethylchloracetat ist und (c) R eine primäre Alkylgruppe mit 8 bis 18 Kohlenstoffatomen ist und R' und R'' unabhängig voneinander aus Methyl und primären Alkylgruppen mit 8 bis 18 Kohlenstoffatomen ausgewählt sind.

10. Verfahren nach Anspruch 1, bei dem ein Halogenalkanoatsalz mit einem tertiärem Amin in einen aprotischen Lösungsmittel zur Umsetzung gebracht wird, in dem das feste Betain im wesentlichen unlöslich ist.

11. Verfahren nach Anspruch 10, bei dem (a) das Halogenalkanoat eine Verbindung der Formel X(CH₂)ₙCOOM ist, in der X Chlor, Brom oder Iod, M Alkalimetall oder Ammonium und n eine ganze Zahl von 1 bis 6 bedeutet, (b) das tertiäre Amin eine Verbindung der Formel RR'R''N ist, in der R eine Alkylgruppe mit 6 bis 22 Kohlenstoffatomen, R' Methyl, Ethyl oder 2-Hydroxyethyl und R'' unabhängig aus Methyl, Ethyl, 2-Hydroxyethyl und Alkylgruppen mit 6 bis 22 Kohlenstoffatomen ausgewählt ist und (c) das Lösungsmittel ein Ester, Keton, Ether, Amid, Nitril oder Sulfoxid ist.

12. Verfahren nach Anspruch 11, bei dem (a) das Halogenalkanoat Natriumchloracetat ist, (b) R eine primäre Alkylgruppe mit 8 bis 18 Kohlenstoffatomen ist und R' und R'' unabhängig voneinander aus Methyl und primären Alkylgruppen mit 8 bis 18 Kohlenstoffatomen ausgewählt sind und (c) das Lösungsmittel ein Ester ist.

13. Verfahren nach Anspruch 1, bei dem ein Halogenalkanoatsalz in einem verflüssigten Gas als Lösungsmittel mit einem tertiären Amin zur Umsetzung gebracht wird.

14. Verfahren nach Anspruch 13, bei dem (a) das Halogenalkanoat eine Verbindung der Formel X(CH₂)ₙCOOM ist, in der X Chlor, Brom oder Iod, M Alkalimetall oder Ammonium und n eine ganze Zahl von 1 bis 6 bedeutet, (b) das tertiäre Amin eine Verbindung der Formel RR'R''N ist, in der R eine Alkylgruppe mit 6 bis 22 Kohlenstoffatomen, R' Methyl, Ethyl oder 2-Hydroxyethyl und R'' unabhängig aus Methyl, Ethyl, 2-Hydroxyethyl und Alkylgruppen mit 6 bis 22 Kohlenstoffatomen ausgewählt ist und (c) das Lösungsmittel ein verflüssigtes Gas mit einer kritischen Temperatur von mindestens 0°C ist.

15. Verfahren nach Anspruch 13, bei dem (a) das Halogenalkanoat Natriumchloracetat ist, (b) R eine primäre Alkylgruppe mit 8 bis 18 Kohlenstoffatomen ist und R' und R'' unabhängig voneinander aus Methyl und primären Alkylgruppen mit 8 bis 18 Kohlenstoffatomen ausgewählt sind und (c) das verflüssigte Gas Kohlendioxid ist.

## Revendications

1. Procédé de préparation d'une bétaïne solide par la réaction de (1) un sel halogénoalcanoate avec une amine tertiaire ou de (2) une base avec l'ester d'ammonium quaternaire résultant de la quaternisation d'une amine tertiaire avec un halogénoalcanoate d'alkyle, caractérisé en ce que la réaction est conduite dans un solvant de gaz liquéfié ou dans un solvant polaire aprotique dans lequel la bétaïne solide est essentiellement insoluble.

2. Procédé selon la revendication 1, dans lequel on fait réagir une base avec l'ester d'ammonium quaternaire résultant de la quaternisation d'une amine tertiaire avec un halogénoalcanoate d'alkyle.

3. Procédé selon la revendication 2, dans lequel la réaction de quaternisation et la réaction avec la base sont toutes deux conduites dans un solvant polaire aprotique dans lequel la bétaïne solide est essentiellement insoluble.

4. Procédé selon la revendication 3, dans lequel l'ester d'ammonium quaternaire que l'on fait réagir avec la base est un ester qui a été récupéré du mélange de sa réaction de synthèse avant d'être mis en contact avec la base.

5. Procédé selon la revendication 3, dans lequel l'amine tertiaire est mise a réagir avec l'halogénoalcanoate de manière à former l'ester d'ammonium quaternaire dans le solvant polaire aprotique, et la base est ensuite ajoutée au mélange résultant, et mise à réagir avec l'ester d'ammonium quaternaire.

6. Procédé selon la revendication 2, dans lequel la réaction de quaternisation et la réaction avec la base sont toutes deux conduites dans un gaz liquéfié servant de solvant.

7. Procédé selon la revendication 2, dans lequel la réaction de quaternisation est conduite dans un solvant de gaz liquéfié, et dans lequel la réaction avec la base est conduite dans un solvant polaire aprotique dans lequel la bétaïne solide est essentiellement insoluble.

8. Procédé selon l'une quelconque des revendications 2-7, dans lequel (a) la base est un hydroxyde d'un métal du groupe IA ou IIA, (b) l'halogénoalcanoate est un composé correspondant à la formule X(CH₂)ₙCOOZ, dans laquelle X représente le chlore, le brome ou l'iode; Z est un radical alkyle contenant 1-6 atome de carbone, et (c) l'amine tertiaire est un composé correspondant à la formule RR'R''N, dans laquelle R est un radical alkyle contenant 6-22 atomes de carbone; R' est le méthyle, l'éthyle ou le 2-hydroxyéthyle; et R'' est choisi indépendamment parmi des radicaux méthyle, éthyle, 2-hydroxyéthyle et alkyle contenant 6-22 atomes de carbone.

9. Procédé selon la revendication 8, dans lequel (a) la base est l'hydroxyde de sodium, (b) l'halogénoalcanoate est le chloroacétate d'éthyle et (c) R est un radical alkyle primaire contenant 8-18 atomes de carbone, et R' et R'' sont choisis indépendamment parmi des radicaux méthyle et alkyle primaire contenant 8-18 atomes de carbone.

10. Procédé selon la revendication 1, dans lequel un sel halogénoalcanoate est mis à réagir avec une amine tertiaire dans un solvant polaire aprotique dans lequel la bétaïne solide est essentiellement insoluble.

11. Procédé selon la revendication 10, dans lequel (a) l'halogénoalcanoate est un composé correspondant à la formule X(CH₂)ₙCOOM, dans laquelle X est le chlore, le brome ou l'iode; M est un métal alcalin ou l'ammonium; et n est un entier valant 1-6, (b) l'amine tertiaire est un composé correspondant à la formule RR'R''N, dans laquelle R est un radical alkyle contenant 6-22 atomes de carbone; R' est le méthyle, l'éthyle ou le 2-hydroxyéthyle; et R'' est choisi indépendamment parmi des radicaux méthyle, éthyle, 2-hydroxyéthyle et alkyle contenant 6-22 atomes de carbone, et (c) le solvant est un ester, une cétone, un éther, une amide, un nitrile ou un sulfoxyde.

12. Procédé selon la revendication 11, dans lequel (a) l'halogénoalcanoate est le chloroacétate de sodium, (b) R est un radical alkyle primaire contenant 8-18 atomes de carbone, et R' et R'' sont choisis indépendamment parmi des radicaux méthyle et alkyle primaire contenant 8-18 atomes de carbone, et (c) le solvant est un ester.

13. Procédé selon la revendication 1, dans lequel on fait réagir un sel halogénoalcanoate avec une amine tertiaire dans un gaz liquéfié servant de solvant.

14. Procédé selon la revendication 13, dans lequel (a) l'halogénoalcanoate est un composé correspondant à la formule X(CH₂)ₙCOOM, dans laquelle X est le chlore, le brome ou l'iode; M est un métal alcalin ou l'ammonium; et n est un entier valant 1-6, (b) l'amine tertiaire est un composé correspondant à la formule RR'R''N, dans laquelle R est un radical alkyle contenant 6-22 atomes de carbone; R' est le méthyle, l'éthyle ou le 2-hydroxyéthyle; et R'' est choisi indépendamment parmi des radicaux méthyle, éthyle, 2-hydroxyéthyle et alkyle contenant 6-22 atomes de carbone, et (c) le solvant est un gaz liquéfié qui présente une température critique d'au moins 0°C.

15. Procédé selon la revendication 13, dans lequel (a) l'halogénoalcanoate est le chloroacétate de sodium, (b) R est un radical alkyle primaire contenant 8-18 atomes de carbone et R' et R'' sont choisis indépendamment parmi des radicaux méthyle et alkyle primaire contenant 8-18 atomes de carbone, et (c) le gaz liquéfié est l'anhydride carbonique.
